(19) Europäisches Patentamt
European Patent Office
Office européen des brevets

(11) **EP 3 740 079 B1**

(12) **EUROPEAN PATENT SPECIFICATION**

(45) Date of publication and mention
of the grant of the patent:
**21.02.2024 Bulletin 2024/08**

(21) Application number: **19700400.5**

(22) Date of filing: **15.01.2019**

(51) International Patent Classification (IPC):
**A23C 9/12** *(2006.01)*  **A23C 9/127** *(2006.01)*

(52) Cooperative Patent Classification (CPC):
**A23C 9/1216; A23C 9/1209; A23C 9/1275;**
C12Y 301/01004; C12Y 301/01032;
C12Y 304/23004

(86) International application number:
**PCT/EP2019/050874**

(87) International publication number:
**WO 2019/138121 (18.07.2019 Gazette 2019/29)**

(54) **FERMENTED MILK PRODUCT AND PREPARATION THEREOF USING PHOSPHOLIPASE**

FERMENTIERTES MILCHPRODUKT UND HERSTELLUNG DAVON UNTER VERWENDUNG VON PHOSPHOLIPASE

PRODUIT DE LAIT FERMENTÉ ET SA PRÉPARATION À L'AIDE D'UNE PHOSPHOLIPASE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.01.2018 EP 18151641**

(43) Date of publication of application:
**25.11.2020 Bulletin 2020/48**

(73) Proprietor: **Chr. Hansen A/S
2970 Hoersholm (DK)**

(72) Inventors:
 • **DI TECCO, Thierry
 Singapore 118259 (SG)**
 • **LOW, Wan Mei
 Singapore 118259 (SG)**
 • **ROUSTEL, Sebastien
 2970 Hoersholm (DK)**

(56) References cited:
WO-A1-03/070013      CN-A- 104 651 391
JP-A- S57 189 638     US-A1- 2008 299 252
US-A1- 2009 285 934

• DATABASE EMBASE [Online] ELSEVIER SCIENCE PUBLISHERS, AMSTERDAM, NL; 1 December 2015 (2015-12-01), WANG H ET AL: "Secretory expression of a phospholipase A2 from Lactobacillus casei DSM20011 in Kluyveromyces lactis", Database accession no. EMB-2015137288 & WANG H ET AL: "Secretory expression of a phospholipase A2 from Lactobacillus casei DSM20011 in Kluyveromyces lactis", JOURNAL OF BIOSCIENCE AND BIOENGINEERING 20151201 ELSEVIER NLD, vol. 120, no. 6, 1 December 2015 (2015-12-01), pages 601-607, ISSN: 1389-1723
• LEE SEUL GI ET AL: "Assessment ofBacillus subtilisSN7 as a starter culture forCheonggukjang, a Korean traditional fermented soybean food, and its capability to controlBacillus cereusinCheonggukjang", FOOD CONTROL, BUTTERWORTH, LONDON, GB, vol. 73, 4 October 2016 (2016-10-04), pages 946-953, XP029850446, ISSN: 0956-7135, DOI: 10.1016/J.FOODCONT.2016.10.015
• Hui Wang ET AL: "Secretory expression of a phospholipase A2 from Lactobacillus casei DSM20011 in Kluyveromyces lactis", Journal of bioscience and bioengineering, vol. 120, no. 6, 1 December 2015 (2015-12-01), pages 601-607, XP055681155, NL ISSN: 1389-1723, DOI: 10.1016/j.jbiosc.2015.03.022

EP 3 740 079 B1

**Description**

FIELD OF THE INVENTION

The present invention generally relates to processes of producing fermented milk products.

BACKGROUND OF THE INVENTION

Milk products such as fermented milk products are known in the art. Texture is a very important quality parameter for fermented milk products. A smooth consistency with good mouthfeel and gel firmness is desired by many consumers.

Addition of protein, typically skim milk powder or whey based proteins, is a standard procedure to improve the texture of fermented milk products. Thickeners or other texturizing agents like modified starch, corn starch, pectin, gelatin, or agar are often used. However, adding proteins or texturizing agents can be costly. It is thus advantageous to provide a process of producing a fermented milk product in which the addition of such agents can be reduced or eliminated. This is desirable since there is an increased market demand for fermented milk products with a 'clean label', i.e. no addition of stabilizing or texturizing agents.

Homogenization is another common procedure to give good texture to fermented milk products. It is performed prior to fermentation to break up the milk fat into smaller sizes. Smaller fat globules created in this process can be easily suspended in solution so it does not exist as a separate layer from the milk. Homogenization can be accomplished for example by forcing the milk through a fine filter or restrictive valve at high pressures, thereby forming an emulsion with decreased particle size. Homogenization pressure normally applied in the dairy industry is around 150 - 250 bar depending on the product. However, homogenization is expensive since it requires a major expenditure of energy. Therefore, it would be advantageous to provide a process in which the need of homogenization is reduced, thereby lowering the operating costs for the manufacturer.

Attempts have been made to address this issue. Many of them relate to providing new strains of lactic acid bacteria that can result in better texture. For example, WO2007/095958A1 (Chr. Hansen) describes using certain starter cultures that produce extracellular polysaccharides to improve the texture of fermented milk products. JPS57189638A discloses a process for preparing an acidified milk product, by treating the milk base with phospholipase A or C before inoculation with lactic acid starter comprising Streptococcus thermophilus and Lactobacillus bulgaricus.

There remains a constant need in the art to improve the sensory properties of fermented milk products, in particular the mouthfeel and mouth coating of the products.

SUMMARY OF THE INVENTION

The present invention is based in part on the surprising finding that phospholipases have positive effects on the sensory qualities of fermented milk products. By using phospholipase(s) in the process of preparation it is possible to reduce or even eliminate the use of proteins, texturizing agents or stabilizing agents in the fermented milk products. It is also possible to reduce the homogenization cost required in the manufacturing process.

The present invention provides a novel use of phospholipase in a process for preparing a fermented milk product. Fermented milk product such as yogurt may be produced from milk base that has been standardized with respect to fat and protein content, homogenized and heat treated. Afterwards, the milk is inoculated with a starter culture and fermented.

It has been observed that phospholipase can actively participate in fermentation process by lactic acid bacteria, resulting in an increase in viscosity of the final fermented product. The present invention thus provides a process for producing a fermented milk product in which a phospholipase-containing milk base is fermented. The application provides a process comprising adding a starter culture to a milk base, fermenting the milk base for a period of time until a target pH is reached, wherein at least one phospholipase A is added to the milk base. The phospholipase is added at the start of the fermentation. After a target pH is reached a fermented milk product is obtained.

The present application provides a process for producing a fermented milk product comprising the steps of:

a) adding a starter culture comprising at least one lactic acid bacteria strain to a milk base characterized by a fat content of at least 1.5% (w/w),

b) fermenting the milk base for a period of time until a target pH is reached, wherein the target pH is 5.0 or lower, and

c) adding at least one phospholipase A to the milk base at the start of the the fermentation period.

The phospholipase which may be used for the present application includes phospholipase A such as phospholipase A1 (EC 3.1.1.32) and phospholipase A2 (EC 3.1.1.4),

Included in the present application is a fermented milk product obtained by the processes described herein. In another aspect, the present invention provides a fermented milk product comprising phospholipase.

In a further aspect, the present invention provides a kit comprising a frozen or freeze-dried direct vat set (DVS) starter culture and at least one phospholipase A useful for making fermented milk products.

Other features and advantages of the invention will become apparent from reading the following description in conjunction with the accompanying figures.

BRIEF DESCRIPTION OF THE FIGURES

Figure 1 depicts the hysteresis curve of fermented milk products prepared in example 1 showing shear stress as a function of shear rates.

Figure 2 depicts factor effects on the response variable (viscosity at 60 1/s) in fermented milk products prepared in example 2.

Figure 3 depicts factor effects on the response variable (viscosity at 300 1/s) in fermented milk products prepared in example 2.

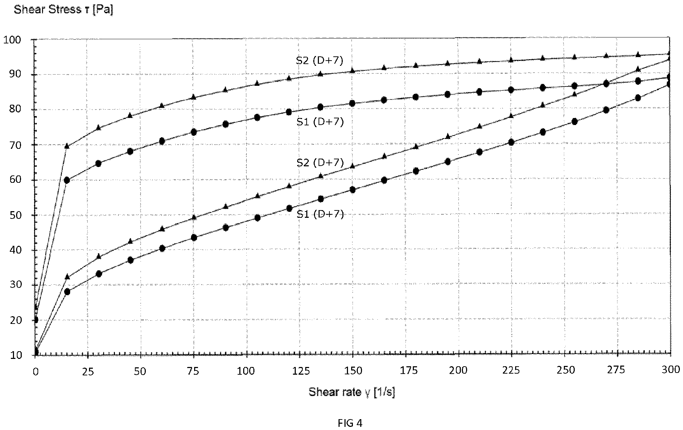

Figure 4 depicts the hysteresis curve of fermented milk products prepared in example 3 showing shear stress as a function of shear rates.

DETAILED DISCLOSURE OF THE INVENTION

This invention relates to fermented milk products produced by fermentation of milk with selected microorganisms. Fermented milks can be characterized by specific starter cultures used in the fermentation. For example, symbiotic cultures of *Streptococcus thermophilus* and *Lactobacillus delbrueckii* subsp. *bulgaricus* are used as starter culture for yogurt, whereas *Lactobacillus acidophilus* is used to make acidophilus milk. Other mesophilic lactic acid bacteria are used to produce quark or fromage frais.

To prepare products in accordance with the present application, a milk base is first provided as starting material. "Milk base" is broadly used in the present application to refer to a composition based on milk or milk components which can be used as a medium for growth and fermentation of a starter culture. "Milk" generally refers to the lacteal secretion obtained by milking of any mammal, such as cows, sheep, goats, buffaloes or camels. Milk base can be obtained from any raw and/or processed milk material as well as from reconstituted milk powder. Milk base can also be plant-based, i.e. prepared from plant material e.g. soy milk.

Useful milk bases include, but are not limited to, solutions/suspensions of any milk or milk like products comprising protein, such as whole or low fat milk, skim milk, buttermilk, reconstituted milk powder, condensed milk, dried milk.

In the methods and products of the present invention, milk base prepared from milk or milk components from cows is preferred.

Milk base may also be lactose-reduced depending on the need of the consumers. Lactose-reduced milk can be produced according to any method known in the art, including hydrolyzing the lactose by lactase enzyme to glucose and galactose, or by nanofiltration, electrodialysis, ion exchange chromatography and centrifugation.

Pasteurization

Milk base is preferably pasteurized prior to fermentation according to methods known in the art. "Pasteurizing" as used herein means the treatment of the milk base to reduce or eliminate the presence of live organisms such as microorganisms. Preferably, pasteurization is attained by maintaining a specified temperature for a specified period of time. The specified temperature is usually attained by heating. Those of ordinary skill in the art are capable of selecting temperature and duration to kill or inactivate certain microorganisms. A rapid cooling step may follow.

Homogenization

"Homogenizing" refers to the process of homogenizing a mixture to obtain a uniform liquid composition out of non-miscible components. The homogenization process breaks up the milk fat into particles of a smaller size so it no longer separates from the milk. This may be accomplished by forcing the milk at high pressure through small orifices. Homogenization is normally applied in the dairy industry after or at the same time as pasteurization. As will become apparent in the following description, the present invention provides processes in which the need of homogenization is reduced. Thus, in some embodiments the milk base is homogenized though at a lower pressure than normally required, and in other embodiments the milk base is not homogenized.

## Fermentation

To ferment the milk base a starter culture is added. The term "starter" or "starter culture" as used in the present context refers to a culture of one or more food-grade microorganisms in particular lactic acid bacteria, which are responsible for the acidification of the milk base. Starter cultures may be fresh, frozen or freeze-dried. It is within the skills of ordinary practitioners to determine the starter culture and amounts to be used.

"Fermentation" generally means the conversion of carbohydrates into alcohols or acids through the action of a microorganism. In the present invention, fermentation in the methods of the invention refers to the conversion of lactose to lactic acid.

## Starter culture

In accordance with the present invention the starter culture comprises at least one strain of lactic acid bacteria. Lactic acid bacteria are extensively used for production of fermented foods and their use is well known in the art. In the context of the present application, the term "lactic acid bacteria" or "LAB" is used to refer to food-grade bacteria producing lactic acid as the major metabolic end-product of carbohydrate fermentation. These bacteria are related by their common metabolic and physiological characteristics and are usually Gram-positive, low-GC, acid tolerant, non-sporulating, non-respiring, rod-shaped bacilli or cocci. During the fermentation stage, the consumption of lactose by these bacteria causes the formation of lactic acid, reducing the pH and leading to the formation of a protein coagulum.

A starter culture useful for the present invention may have the strain composition of any conventional starter culture of lactic acid bacteria, including single strain culture or culture blends, depending on the specific type of fermented milk product. Other useful bacteria, including the probiotic bacteria *Bifidobacterium* spp, may also be included in the fermentation in addition to the starter culture.

In one embodiment, the starter culture comprises thermophilic bacteria to produce thermophilic fermented milk product. The term "thermophilic fermented milk product" refers to fermented milk products prepared by thermophilic fermentation of a thermophilic starter culture and examples include fermented milk products such as set-yogurt, stirred-yogurt and drinking yogurt, e.g. Yakult. Industrially, the most useful thermophilic bacteria ("thermophiles") include *Streptococcus* spp. and *Lactobacillus* spp. The term "thermophilic fermentation" herein refers to fermentation at a temperature above about 35°C, such as between about 35°C and about 45°C. "Thermophiles" are generally microorganisms that thrive best at temperatures above 35°C.

In another embodiment the starter culture comprises mesophilic bacteria to produce mesophilic fermented milk product. The term "mesophilic fermented milk product" refers to fermented milk products prepared by mesophilic fermentation of a mesophilic starter culture and examples include fermented milk products such as buttermilk, sour milk, cultured milk, smetana, sour cream, kefir and fresh cheese, such as quark, tvarog and cream cheese. Useful mesophilic bacteria ("mesophiles") include *Lactococcus* spp. and *Leuconostoc* spp. The term "mesophilic fermentation" herein refers to fermentation at a temperature between about 22°C and about 37°C. The term "mesophiles" generally refers to microorganisms which thrive best at moderate temperatures (15°C-35°C).

Lactic acid bacteria useful for making fermented milk product encompasses, but is not limited to, bacteria belonging to the genus of *Lactobacillus spp., Bifidobacterium spp., Streptococcus* spp., *Lactococcus* spp., such as *Lactobacillus delbrueckii* subsp. *bulgaricus, Streptococcus thermophilus, Lactobacillus lactis, Bifidobacterium animalis, Lactococcus lactis, Lactobacillus paracasei, Lactobacillus plantarum, Lactobacillus helveticus, Lactobacillus acidophilus, Lactobacillus fermentum, Lactobacillus rhamnosus, Bifidobacterium breve* and *Leuconostoc* spp. The specific selection of strains in the starter culture will depend on the particular type of fermented dairy product to be manufactured.

In a preferred embodiment the starter culture contains at least one *Lactobacillus delbrueckii* subsp. *bulgaricus* strain and at least one *Streptococcus thermophilus* strain.

In a preferred embodiment, the lactic acid bacteria are selected from the group consisting of bacteria from the genera *Lactobacillus, Streptococcus, Lactococcus,* and *Leuconostoc.*

In a particular embodiment of the invention, the fermented milk product is a product obtained using one or more lactic acid bacteria strain selected from the group consisting of *Streptococcus thermophilus* and *Lactobacillus delbrueckii* subsp. *bulgaricus.*

## Process of fermentation

After adding the starter culture and subjecting the milk base to a suitable condition, the fermentation process begins and continues for a period of time. A person of ordinary skill in the art knows how to select suitable process conditions, such as temperature, oxygen, addition of carbohydrates, amount and characteristics of microorganism(s) and the process time it takes. This process may take from three, four, five, six hours or longer.

These conditions include the setting of a temperature which is suitable for the particular starter culture strains. For example, when the starter culture comprises mesophilic lactic bacteria, the temperature can be set to about 30°C, and if the culture comprises thermophilic lactic acid bacterial strains, the temperature is kept in the range of about 35°C to 50°C, such as 40°C to 45°C. The setting of the fermentation temperature also depends on the enzyme(s) added to the fermentation which can be readily determined by a person of ordinary skill in the art. In a particular embodiment of the invention the fermentation temperature is between 35°C and 45°C, preferably between 37°C and 43°C, and more preferably between 40°C and 43°C.

Fermentation can be terminated using any methods known to in the art. In general, depending on various parameters of the process, the fermentation can be terminated by making the milk base unsuitable for the strain(s) of the starter culture to grow. For example, termination can be carried out by rapid cooling of the fermented milk product when a target pH is reached. It is known that during fermentation acidification occurs, which leads to the formation of a three-dimensional network consisting of clusters and chains of caseins. The term "target pH" means the pH at which the fermentation step ends. The target pH depends on the fermented milk product to be obtained and can be readily determined by a person of ordinary skill in the art.

In a particular embodiments of the invention, fermentation is carried out until a pH of 5.0 is reached, including until a pH of 4.9, 4.8, 4.7, 4.6, 4.5, 4.4, 4.3, 4.2, 4.1, 4.0, 3.9, 3.8 or 3.7 is reached. Preferably, the fermentation is carried out until a target pH between 4.0 and 5.0 and more preferably between 4.0 and 4.6 is reached. In a preferred embodiment, the fermentation is carried out until target pH below 4.6 is reached.

Phospholipase

Milk comprises phospholipids. The phospholipids are associated with the milk fat due to its non-polar, lipophilic properties. Phospholipids such as lecithin or phosphatidylcholine consist of glycerol esterified with two fatty acids in an outer (sn-1) and the middle (sn-2) positions and esterified with phosphoric acid in the third position. Phosphoric acid, in turn, may be esterified to an amino-alcohol. Phospholipids may be hydrolyzed by phospholipase into lysophospholipid, which may in turn be hydrolyzed by a lysophospholipase.

Phospholipases are fundamental enzymes that play a crucial role in living organisms in general and in the metabolism and biosynthesis of phospholipids in particular. The enzymes participate in the hydrolysis of phospholipids and several types of phospholipase activity can be distinguished. Phospholipase A can further be classified as phospholipase A1 (EC 3.1.1.32) or A2 (EC 3.1.1.4.), which hydrolyze one fatty acyl group (in the sn-1 and sn-2 position, respectively) to form lysophospholipid. Phospholipase B (EC 3.1.1.5) hydrolyzes the remaining fatty acyl group in lysophospholipid. Other phospholipases are phospholipase C (EC 3.1.4.3) and phospholipase D (EC 3.1.4.4).

The phospholipase(s) useful for the present application is phospholipase A. More preferably, the phospholipase is phospholipase A1 (EC 3.1.1.32) and/or phospholipase A2 (EC 3.1.1.4).

Phospholipase A1 is defined according to standard enzyme EC-classification as EC 3.1.1.32.

Official Name: Phospholipase A1

Reaction catalyzed: phosphatidylcholine + water < = > 2-acylglycerophosphocholine + a fatty acid anion

Comment: has a much broader specificity than EC 3.1.1.4.

Phospholipase A2 is defined according to standard enzyme EC-classification as EC 3.1.1.4

Official Name: Phospholipase A2.

Alternative Names: phosphatidylcholine 2-acylhydrolase. lecithinase a; phosphatidase; or phosphatidolipase.

Reaction catalyzed: phosphatidylcholine + water < = > i-acylglycerophosphocholine + a fatty acid anion

Comment: also acts on phosphatidylethanolamine, choline plasmalogen and phosphatides, removing the fatty acid attached to the 2-position.

The phospholipase may be of any origin, e.g. of animal origin (such as, e.g. mammalian), e.g. from pancreas (e.g. bovine or porcine pancreas), or snake venom or bee venom. Alternatively, the phospholipase may be of microbial origin, e.g. from filamentous fungi, yeast or bacteria, such as the genus or species *Aspergillus,* e.g. *A. niger; Dictyostelium,* e.g. *D. discoideum; Mucor,* e.g. *M. javanicus, M. mucedo, M. subtilissimus; Neurospora,* e.g. *N. crassa; Rhizomucor,* e.g. *R. pusillus; Rhizopus,* e.g. *R. arrhizus, R. japonicus, R. stolonifer; Sclerotinia,* e.g. *S. libertiana; Trichophyton,* e.g. *T. rubrum; Whetzelinia,* e.g. *W. sclerotiorum; Bacillus,* e.g. *B. megaterium, B. subtilis; Citrobacter,* e.g. *C. freundii; Enterobacter,* e.g. *E. aerogenes, E. cloacae Edwardsiella, E. tarda; Erwinia,* e.g. *E. herbicola; Escherichia,* e.g. *E. coli; Klebsiella,* e.g. *K. pneumoniae; Proteus,* e.g. *P. vulgaris; Providencia,* e.g. *P. stuartii; Salmonella,* e.g. *S. typhimurium; Serratia,* e.g. *S. liquefasciens, S. marcescens; Shigella,* e.g. *S. flexneri; Streptomyces,* e.g. *S. violeceoruber; Yersinia,* e.g. *Y. enterocolitica.* The phospholipase may be fungal, e.g. from the class *Pyrenomycetes,* such as the genus *Fusarium,* such as a strain of *F. culmorum, F. heterosporum, F. solani, F. venenatum,* or a strain of *F. oxysporum.* The phospholipase may also be from a filamentous fungus strain within the genus *Aspergillus,* such as a strain *of Aspergillus awamori, Aspergillus foetidus, Aspergillus japonicus, Aspergillus niger* or *Aspergillus oryzae.* A preferred phospholipase is derived from a strain of *Fusarium,* particularly from *F. venenatum* or *F. oxysporum,* e.g. from strain DSM 2672 as described in WO 98/26057, especially described in claim 36 of WO 98/26057. In further embodiments, the phospholipase is a phospholipase as disclosed in WO 00/32758 (Novozymes A/S, Denmark). Another preferred phospholipase A is phospholipase A2 from Streptomyces, such as e.g. PLA2 from *S. violaceoruber.*

Phospholipases are commercially available. Phospholipase A1 from *Thermomyces lanuginosus/Fusarium oxysporum* expressed in *Aspergillus oryzae* is available under the trademark Lecitase®Ultra (Novozymes A/S, Denmark). Another preferred phospholipase A1 is available under the trademark YieldMAX® or YieldMAX®PL (Chr. Hansen A/S, Denmark), which is produced from submerged fermentation of an *Aspergillus oryzae* strain.

A particularly preferred phospholipase is phospholipase A1 from *Fusarium* spp.

Phospholipase A2 can be found under different trade names. For example. phospholipase A2 from animal origin (porcine pancreas) developed for the degumming of vegetable oils is available under Lecitase®10L (Novozymes A/S, Denmark). Maxapal® A2 (DSM Food Specialties, The Netherlands) is produced by microbial fermentation of a selected strain of *Aspergillus niger* developed to improve emulsifying properties of egg and egg yolk. CakeZyme® and BakeZyme® are also microbial phospholipase A2 commercialized by DSM Food Specialties (The Netherlands). Further examples include microbial phospholipase A2 Rohalase®MPL (AB Enzymes, Germany) and LysoMax® (Genencor, USA).

In a preferred embodiment, the phospholipase is YieldMAX® or YieldMAX®PL (Chr. Hansen A/S, Denmark). Other food-grade phospholipases are well known to a skilled person in the art and can be found for example in Casado, Victor, et al. "Phospholipases in food industry: a review." Lipases and Phospholipases: Methods and Protocols (2012): 495-523.

In some embodiments, phospholipase(s) used in the process of the invention is derived or obtainable from any of the sources mentioned herein. The term "derived" means in this context that the phospholipase may have been isolated from an organism where it is present natively, i.e. the identity of the amino acid sequence of the enzyme are identical to a native enzyme. The term "derived" also means that the enzymes may have been produced recombinantly in a host organism, the recombinant produced enzyme having either an identity identical to a native enzyme or having it a modified amino acid sequence, e.g. having one or more amino acids which are deleted, inserted and/or substituted, i.e. a recombinantly produced enzyme which is a mutant and/or a fragment of a native amino acid sequence. Within the meaning of a native enzyme are included natural variants. Furthermore, the term "derived" includes enzymes produced synthetically by e.g. peptide synthesis. The term "derived" also encompasses enzymes which have been modified e.g. by glycosylation, phosphorylation etc., whether *in vivo* or *in vitro.* The term "obtainable" in this context means that the enzyme has an amino acid sequence identical to a native enzyme. The term encompasses an enzyme that has been isolated from an organism where it is present natively, or one in which it has been expressed recombinantly in the same type of organism or another, or enzymes produced synthetically by e.g. peptide synthesis. With respect to recombinantly produced enzyme the terms "obtainable" and "derived" refers to the identity of the enzyme and not the identity of the host organism in which it is produced recombinantly.

Accordingly, the phospholipase may be obtained from a microorganism by use of any suitable technique. For instance, a phospholipase enzyme preparation may be obtained by fermentation of a suitable microorganism and subsequent isolation of a phospholipase from the resulting fermented broth or microorganism by methods known in the art. The phospholipase may also be obtained by use of recombinant DNA techniques. Such method normally comprises cultivation of a host cell transformed with a recombinant DNA vector comprising a DNA sequence encoding the phospholipase in question and the DNA sequence being operationally linked with an appropriate expression signal such that it is capable of expressing the phospholipase in a culture medium under conditions permitting the expression of the enzyme and recovering the enzyme from the culture. The DNA sequence may also be incorporated into the genome of the host cell. The DNA sequence may be of genomic, cDNA or synthetic origin or any combinations of these, and may be isolated or synthesized in accordance with methods known in the art. Such phospholipase may be purified. The term "purified" as used herein covers phospholipase enzyme free from components from the organism from which it is derived. The term "purified" also covers phospholipase enzyme free from components from the native organism from which it is obtained, this is also termed "*essentially* pure" phospholipase and may be particularly relevant for phospholipases which are naturally occurring and which have not been modified genetically, such as by deletion, substitution or insertion of one or more amino acid residues. Purification can be done by filtration, precipitation, or chromatography for instance.

Phospholipases used in the present application can be in any suitable form, such as in the form of a dry powder or granulate, a non-dusting granulate, a liquid, a stabilized liquid, or a protected enzyme. Granulates may be produced, e.g. as disclosed in US Pat No. 4,106,991 and US Pat No. 4,661,452, and may optionally be coated by methods known in the art. Liquid enzyme preparations may, for instance, be stabilized by adding stabilizers such as a sugar, a sugar alcohol or another polyol, lactic acid or another organic acid according to established methods. Protected enzymes may be prepared according to the method disclosed in European Pat. No. 238,216.

The activity of a phospholipase can be determined as the rate of sodium hydroxide consumption during neutralization of the fatty acid. Such activity can be expressed in Lecitase units (LEU) relative to a Lecitase (phospholipase) standard. The procedures are known in the art. For example, phospholipase A1 activity may be measured relative to a phospholipase standard using lecithin as a substrate. Phospholipase A1 catalyzes the hydrolysis of lecithin to lysolecithin and a free fatty acid. The liberated fatty acid is titrated with 0.1 N sodium hydroxide under standard conditions (pH=8.0; 40° ±0.5). 1 LEU is defined as the amount of enzyme that under standard conditions (pH=8.0; 40° ±0.5) results in the same rate of sodium hydroxide consumption (in microeq/min) as the Lecitase standard diluted to a nominal activity of 1 LEU/g. The method can be carried out using either an automated system or standard laboratory equipment for carrying out titration experiments.

Addition of phospholipase

Phospholipase is added to the milk base at the start of the fermentation. The expression "at the start of the fermentation" means shortly before, at the same time as, or shortly after addition of the starter culture to the milk base. Here, the term "shortly" means less than 30 minutes.

The phospholipase is added to the milk base at the start of the fermentation step. The fermentation step may be terminated by cooling treatment.

The amount of phospholipase to be added to the milk base depends on a number of parameters, including the phospholipase activity and the composition of the milk base such as the fat content, etc. Such amount can be determined by routine experimentation.

Suitable conditions for performing the treatment of phospholipase can be determined by a skilled person using methods known in the art for optimizing enzymatic reactions. The skilled person will know how to adjust parameters such as pH, temperature, and amount of phospholipase to achieve the desired results, taking into consideration the properties desired in the fermented milk product. The amount of phospholipase to be used in the method of the invention may depend on the activity of the specific phospholipase on the phospholipids present under the specific treatment conditions. For instance, when a phospholipase A is used as shown in the example, the amount of phospholipase added may be between 0.1 and 50 LEU per gram of fat, such as between 0.5 and 25, or between 1 and 10 LEU per gram of fat.

Fermented Milk Products

The term "fermented milk product" is a term generally defined in accordance with relevant official regulations and the standards are well known in the field. The expression "fermented milk product" means a food or feed product wherein the preparation of the food or feed product involves fermentation of a milk base with a lactic acid bacterium. "Fermented milk product" as used herein includes but is not limited to products such as thermophilic fermented milk products (e.g. yogurt) and mesophilic fermented milk products (e.g. sour cream and buttermilk, as well as fermented whey, quark and fromage frais). In contrast to cheese products, fermented milk products generally have lower target pH at the end of fermentation.

More particularly, the process as disclosed herein can be applied to obtain thermophilic fermented milk products like yogurt and particularly set-yogurt and mesophilic fermented milk such as quark.

In one embodiment of the invention, the fermented milk product is a yogurt product. The term "yogurt" has its usual meaning and is generally defined in accordance with relevant official regulations and standards are well known in the field. A yogurt product can be selected from the group consisting of "stirred-type product", "set-type product" or "drinkable product."

"Stirred type product" are fermented milk product which sustains a mechanical treatment after fermentation. The mechanical treatment is typically but not exclusively obtained by stirring, pumping, filtrating or homogenizing the gel, or by mixing it with other ingredients. "Set-type product" is a product based on milk which has been inoculated with a starter culture, e.g. a starter culture, and packaged and fermented in the package. The term "drinkable product" includes beverages such as "drinking yogurt", "diluted drinking yogurt" or similar, which may be a milk product produced by fermentation by the combination of *Lactobacillus* species and *Streptococcus thermophilus*.

In a preferred embodiment, fermented milk product is selected from the group consisting of quark, cream cheese, fromage frais, greek yogurt, soy yogurt, skyr, labneh, butter milk, sour cream, sour milk, cultured milk, kefir, lassi, ayran, twarog, doogh, smetana, yakult and dahi. More preferably, the fermented milk product is sour milk, cultured milk, kefir, lassi, ayran, doogh, yakult or dahi.

Protein content

Fermented milk products produced by the process as disclosed herein may contain protein at a level of between 0.5% by weight to 10% by weight. The fermented milk product may also be a low protein product with a protein level between 1% by weight and 4.0% by weight. Alternatively, the fermented milk product may be a high protein product with a protein level of above 3.5% by weight.

In a particular embodiment of the invention, the milk base is characterized by a protein content (w/w) of less than 0.5%, less than 0.7%, less than 0.9%, less than 1.0%, less than 1.3%, less than 1.5%, less than 2.0%, less than 2.5%, less than 3.0%, less than 3.5%, less than 4.0%, less than 4.5%, less than 5.0%, less than 5.5%, less than 6.0%, less than 6.5%, less than 7.0%, less than 7.5%, less than 8.0%, less than 8.5%, less than 9.0%, less than 9.5%, less than 10.0%, less than 10.5%, less than 11.0%, less than 11.5%, less than 12.0%, less than 12.5%, less than 13.0%, less than 13.5%, less than 14.0%, less than 14.5%, or less than 15.0%.

Fat content

The inventors of the present application have discovered the positive impacts of phospholipases on the texture of fermented milk products. It has been observed that the impact is bigger when the fat level of the milk base is higher, making it especially suitable for use in fermenting products containing higher fat level.

In particular embodiments of the invention, the milk base is characterized by a fat content (w/w) of at least 1.5%, at least 2.0%, at least 2.5%, at least 3.0%, at least 3.5%, at least 4.0%, at least 4.5%, at least 5.0%, at least 5.5%, at least 6.0%, at least 6.5%, at least 7.0%, at least 7.5%, at least 8.0%, at least 8.5%, at least 9.0%, at least 9.5%, at least 10.0%, at least 10.5%, at least 11.0%, at least 11.5%, at least 12.0%, at least 12.5%, at least 13.0%, at least 13.5%, at least 14.0%, at least 14.5%, or at least 15.0%. In some embodiments the fat content is at least 16.0%, at least 17.0%, at least 18.0%, at least 19.0%, at least 20.0%, at least 21.0%, at least 22.0%, at least 23.0%, at least 24.0%, at least 25.0%, at least 26.0%, at least 27.0%, at least 28.0%, at least 29.0%, at least 30.0%, at least 35.0%, at least 40.0%, at least 45.0%, at least 50.0% or higher. The adjustment of fat content in a milk base is known to a skilled person in the art, for example by adding cream to the milk base.

In preferred embodiments, the milk base is characterized by a fat content of from 3.0% to 7.0%, preferably from 4.0% to 6.0%, and more preferably from 4.5% to 5.5%. It has been found that the effect of phospholipase(s) on the texture is higher when the fat content is higher.

Homogenization

Following the teaching of the present invention it is possible to provide fermented milk products where the homogenization level usually required for such products is reduced or even eliminated, thereby lowering the operating costs for the manufacturer. Thus, in one embodiment the milk base used for preparing such product is not homogenized. However, in some preferred embodiment, depending on the types of fermented milk product, the milk base may still be homogenized. The homogenization level is preferably under 250 bar, including less than 240 bar, less than 220 bar, less than 200 bar, less than 190 bar, less than 180 bar, less than 170 bar, less than 160 bar, less than 150 bar, less than 140 bar, less than 130 bar, less than 120 bar, less than 110 bar, less than 100 bar, less than 90 bar, less than 80 bar, less than 70 bar, less than 60 bar, less than 50 bar. In the context of the present application, if homogenization is carried out in multiple stages, the homogenization level refers to the total pressure applied in all stages. For example, if the homogenization is carried out in two stages, first at 120 bar and second at 60 bar, the homogenization level of the milk base is 180 bar.

Chymosin

The inventors of the present application have also discovered that the further addition of chymosin (EC 3.4.23.4) can advantageously improve the texture of the fermented milk product. Chymosin is an aspartic protease belonging to a broad class of peptidases and is commonly used in the manufacturing of cheese as a milk-clotting enzyme.

Bovine chymosin, in particular calf chymosin, is commercially available both as stomach enzyme extracts (rennets; comprising the natively produced chymosin) and as recombinantly produced chymosin (which is typically expressed in bacterial, yeast or fungal host cells) (see e.g. WO 95/29999). Other non-bovine chymosins such as *Camelus dromedarius* chymosin or variants thereof have been described in WO2002036752, WO2013174840 and WO2015/128417. Preferred chymosins include commercially available chymosins such as CHY-MAX®, CHY-MAX® M and CHY-MAX®Plus (Chr. Hansen A/S Denmark).

In a preferred embodiment, the process additionally comprises the step of adding a chymosin to the milk base before, at the start, or during the fermentation period. The amount of chymosin to be added to the milk base depends on a number of parameters, including the chymosin activity, the composition of the milk base such as the protein content like casein content, etc. Suitable conditions can be selected by the skilled person in the art and according to methods known in the art for optimizing enzymatic reactions. Assays for determining chymosin activity are known in the art. It can be measured according to a standardized method and expressed in International Milk Clotting Units per gram (IMCU/g).

In one preferred embodiment, the process for producing a fermented milk product comprises adding a starter culture with at least one lactic acid bacteria strain to a milk base, fermenting the milk base for a period of time until a target pH is reached, wherein phospholipase and chymosin are added at the same time or one after another to the milk base at the start of the fermentation period.

Storage

The fermented milk product is preferably stored for at least two days, for example at least 3 days, at least 4 days, more preferably at least 5 days, at least 6 days, at least 7 days, at least 8 days, at least 9 days, at least 10 days, at least 11 days, at least 12 days, at least 13 days, and at least 14 days. In fresh dairy business, products typically reach the consumer within around 3 to 5 days, and generally no later than 7 days after production.

As used herein, the term "storage" or "stored" refers to the holding of products under suitable conditions until they are dispatched to the consumers for consumption. Such conditions are known in the industry and can be determined by a skilled practitioner.

Kit

In another aspect, the present invention provides a kit comprising a starter culture and one or more phospholipase(s). The starter culture comprises one or more lactic acid bacteria useful for producing fermented milk product as mentioned in the present application. Lactic acid bacteria contained in the kit may for example include bacteria *Lactobacillus* spp. and *Lactococcus* spp. They are in the form of frozen or freeze-dried cultures for bulk starter propagation or as so-called "Direct Vat Set" (DVS) cultures intended for direct inoculation into a fermentation vessel or vat for the production of a fermented milk product. The phospholipase included in the kit may be phospholipase A, phospholipase A1, phospholipase A2, or a combination thereof. The kit may further comprise one or more chymosin(s).

Texture of Fermented Milk Products

The present invention also provides a novel use of phospholipase for improving the texture of fermented milk products. Texture may be characterized with respect to rheological properties using methods are well known in the art, including measuring the "shear stress (viscosity)" or "gel firmness" of the product. The SI unit for shear stress and gel firmness is pascal (Pa). It has been observed by the inventors that both gel firmness and viscosity may be improved.

In particular embodiments the use allows for an increase of the viscosity of fermented milk product such as yogurt measured at 13°C at a shear rate of 60 1/s after 7 days of storage.

The term "shear stress" determines viscosity. Viscosity (unit is Pas) is defined as "Shear Stress" (Pa)/Shear rate (1/s). Shear stress value can be reported at different points. Sensory experiments have shown that the best correlations between rheological measurements and sensory are found when viscosity is measured at a shear rate of 60 1/s (correlating well with the first impression in mouth) and at a shear rate of 300 1/s (cohesiveness correlating with difficulty to swallow).

"Gel firmness" of fermented milk products can be measured by a so-called frequency sweep, measuring the complex modulus (G*) of the gel as function of oscillation frequency. The value of G* at 1.52 Hz can be used as the "gel firmness" of the product.

The viscosity of the product can be measured by a so-called constant rate measurement, measuring the shear stress of the product as function of shear rate.

Fermented milk product produced in accordance with the teaching from the present invention may achieve an increase in the viscosity or gel firmness compared to a control product, which is a fermented milk product produced the same way but without using phospholipase, measured at shear rate of 60 1/s or 300 1/s.

In one embodiment, when measured as shear stress in 60 1/s (Pa), the use of phospholipase generates a viscosity in a fermented milk product which is at least about 5% higher, at least about 10% higher, at least about 15% higher, at least about 20% higher, at least about 25% higher, at least about 30% higher, at least about 35% higher, at least about 40% higher, at least about 50% higher, than the viscosity generated by a product where phospholipase is not used.

In another embodiment, when measured as shear stress in 300 1/s (Pa), the use of phospholipase generates a viscosity in a fermented milk product which is at least about 5% higher, at least about 10% higher, at least about 15% higher, at least about 20% higher, at least about 25% higher, at least about 30% higher, at least about 35% higher, at least about 40% higher than the viscosity generated by a product where phospholipase is not used.

In another embodiment, the use of phospholipase generates a gel firmness (G* at 1.52 Hz) in a fermented milk product which is at least about 5% higher, at least about 10% higher, at least about 15% higher, at least about 20% higher, at least about 25% higher, at least about 30% higher than the gel firmness generated by a product where phospholipase is not used.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. Unless otherwise stated, all exact values provided herein are representative of corresponding approximate values (e.g. all exact exemplary values provided with respect to a particular factor or measurement can be considered to also provide a corresponding approximate measurement, modified by "about", where appropriate). The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

EXAMPLES

The invention described and claimed herein is not to be limited in scope by the specific aspects herein disclosed, since these aspects are intended as illustrations of several aspects of the invention.

In the case of conflict, the present disclosure including definitions will prevail.

Example 1

Yogurt products containing 1.5% or 5.0% fat were prepared. Effect of phospholipase on rheological properties at day 7 and day 30 were evaluated.

Preparation of milk base:

Skim milk power and whole milk powder (Fonterra Co-operative Group Limited, New Zealand) was used to prepare milk base for the following examples.

Table 1 Composition of milk base (w/w)

| Sample | Skim milk powder | Whole milk powder | Water |
|---|---|---|---|
| 1 | 8.60% | 5.30% | 86.10% |
| 2 | 8.60% | 5.30% | 86.10% |
| 3 | 8.10% | 9.00% | 82.90% |
| 4 | 8.10% | 9.00% | 82.90% |

The ingredients were mixed with water with Silverson Mixer at 45°C - 50°C. Hydration time was 20 minutes. The milk base was homogenized with GEA Lab Homogenizer in two stages (first stage 150 bar and second stage 50 bar; in total: 200 bar) and pasteurized at 85°C for 30 min and cooled down.

Starter culture:

The starter culture was composed of *Streptococcus thermophilus* and *Lactobacillus delbrueckii* subsp. *Bulgaricus.*

Phospholipase:

Phospholipase A1 (YieldMAX®PL from Chr. Hansen A/S, Denmark; average activity 2600 LEU/ml, dosage 0.021650% for 5% fat) was added at the start of the fermentation. Fat and protein content was determined using MilkoScan analysis.

Fermentation:

Fermentation was carried out at 43°C for four hours and 50 minutes and end pH of 4.55 ± 0.05 was reached. The fermented product was cooled through a plate heat exchanger post-treated to 23°C -25°C and smoothed with a back pressure valve at a pressure of 1 bar at the outlet of the cooler.
The samples were stored at cold room temperature (5°C-7°C).

Table 2 Composition and fermentation details

| Sample | Protein content | Fat content | Phospholipase | End pH | Fermentation Time |
|--------|-----------------|-------------|---------------|--------|-------------------|
| 1 | 4.00% | 1.50% | -- | 4.51 | 4 hours 50 mins |
| 2 | 4.00% | 1.50% | 0.00345% | 4.49 | 4 hours 50 mins |
| 3 | 4.00% | 5.00% | -- | 4.51 | 4 hours 50 mins |
| 4 | 4.00% | 5.00% | 0.01265% | 4.49 | 4 hours 50 mins |

Samples stored at day 7 and day 30 were tested for viscosity and gel firmness.

Measurement of Shear Stress and Gel firmness:

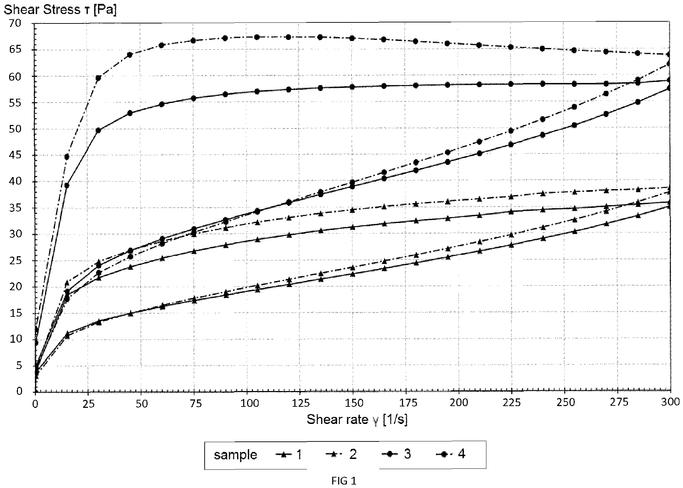

The rheological properties of the sample were assessed on a rheometer (Anton Paar Modular Compact Rheometer MCR 302, Anton Paar® GmbH, Austria). The rheometer was set to a constant temperature of 13°C during the time of measurement. The program Stirred_oscillation+Up_DownFlow was used.
The shear stress at 60 1/s at 300 1/s was chosen for analysis, as this correlates to texture in mouth (mouthfeel, first impression) and cohesiveness (when swallowing a fermented milk product).
Gel firmness (Complex modulus G* at 1.52) was assessed on a rheometer (Anton Paar Modular Compact Rheometer MCR 302, Anton Paar® GmbH, Austria).
Results are given below and hysteresis curve is shown in Figure 1.

Table 3 Rheological assessment of samples at day 7

| Sample | Mouthfeel, first impression (viscosity at 60 1/s) | Cohesiveness Difficulty to swallow (viscosity at 300 1/s) |
|--------|---------------------------------------------------|------------------------------------------------------------|
| 1 | 422.71 | 119.44 |
| 2 | 475.66 | 128.50 |
| 3 | 907.30 | 196.31 |
| 4 | 1092.9 | 212.45 |

Table 4 Rheological assessment of samples at day 30

| Sample | Mouthfeel, first impression (viscosity at 60 1/s) | Cohesiveness Difficulty to swallow (viscosity at 300 1/s) |
|--------|---------------------------------------------------|------------------------------------------------------------|
| 1 | 412.25 | 110.38 |
| 2 | 495.85 | 128.72 |

(continued)

| Sample | Mouthfeel, first impression (viscosity at 60 1/s) | Cohesiveness Difficulty to swallow (viscosity at 300 1/s) |
|---|---|---|
| 3 | 966.16 | 202.41 |
| 4 | 1078.80 | 207.96 |

As shown in Table 3 and 4, samples treated with phospholipase (sample 2 and 4) were shown to have improved viscosity at 60 1/s (mouthfeel, first impression) and at 300 1/s (cohesiveness) compared to the control samples (sample 1 and 3). Effects were stronger in sample with higher fat content (sample 4).

Example 2

1. Experimental setup

A factorial-fractional design was chosen to check the following 5 factors in 16 trials. The objective is to confirm the effect of phospholipases on the texture in a bigger design and to check significance, interaction with homogenization pressure level, and interaction with a chymosin enzyme as well as interaction with starter cultures.

Table 5 Experimental setup: factors

| Factors were coded orthogonally: | | |
|---|---|---|
| Factors | -1 | +1 |
| 1-Homogenization pressure | 150 bars | 300 bars |
| 2-Starter culture | 1 | 2 |
| 3-Protein content | 2.50% | 4.50% |
| 4-Chymosin | no | yes |
| 5-Phospholipase | no | yes |

Table 6 Experimental setup: design

| Trial number | Factor 1 Homogenization pressure | Factor 2 Starter culture | Factor 3 Protein content | Factor 4 Chymosin | Factor 5 Phospholipase |
|---|---|---|---|---|---|
| 1 | - | - | - | - | + |
| 4 | + | + | - | - | + |
| 6 | + | - | + | - | + |
| 7 | - | + | + | - | + |
| 2 | + | - | - | + | + |
| 3 | - | + | - | + | + |
| 5 | - | - | + | + | + |
| 8 | + | + | + | + | + |
| 9 | - | - | - | + | - |
| 10 | + | - | - | - | - |
| 11 | - | + | - | - | - |
| 12 | + | + | - | + | - |
| 13 | - | - | + | - | - |
| 14 | + | - | + | + | - |
| 15 | - | + | + | + | - |

(continued)

| Trial number | Factor 1 Homogenization pressure | Factor 2 Starter culture | Factor 3 Protein content | Factor 4 Chymosin | Factor 5 Phospholipase |
|---|---|---|---|---|---|
| 16 | + | + | + | - | - |

Table 7 Aliasing Structure

| 1 | 2345 |
|---|---|
| 2 | 1345 |
| 3 | 1245 |
| 4 | 1235 |
| 12 | 345 |
| 13 | 245 |
| 14 | 235 |
| 23 | 145 |
| 24 | 135 |
| 34 | 125 |
| 123 | 45 |
| 124 | 35 |
| 134 | 25 |
| 234 | 15 |
| 1234 | 5 |

The fifth factor is studied on an interaction of the third order (5=1234). Therefore all first order interactions can be estimated with good accuracy, considering that second order interaction have a high probability to be very small or null.

2. Procedure

Yogurt products containing 5.0% fat were prepared with varied parameters.

Preparation of milk base:

Skim milk power and cream powder (Fonterra Co-operative Group Limited, New Zealand) were used to prepare the following milk base.

Table 8 Composition of milk base 1 (Protein = 2.50%, Fat = 5.00%)

| Ingredient | Dosage (%) |
|---|---|
| Skim Milk Powder | 3.39 |
| Cream Powder | 9.07 |
| Water | 87.54 |

Table 9 Composition of milk base 2 (Protein = 4.50%, Fat = 5.00%)

| Ingredients | Dosage (%) |
|---|---|
| Skim milk powder | 9.68 |

(continued)

| Ingredients | Dosage (%) |
|---|---|
| Cream powder | 8.98 |
| Water | 81.34 |

Skim milk powder, cream powder and water were mixed with Silverson Mixer at 8°C -10°C for at least 2 hours. The milk base was homogenized with GEA Lab Homogenizer. Depending on the setup, homogenization at 150 bar (first stage 120 bar and second stage 30 bar) or 300 bar (first stage 240 bar and second stage 60 bar) were carried out. The homogenized milk base was then pasteurized at 85°C for 30 min and cooled down.

Preparation and Fermentation:

Depending on the setup, phospholipase and/or chymosin was added at the start of the fermentation together with the starter culture.

Starter Culture:

The starter culture from Example 1 was used (starter culture 1, level-1). A second starter culture (starter culture 2, level+1) with different fermentation kinetics was included for comparison. Both starter cultures were composed of *Streptococcus thermophilus* and *Lactobacillus delbrueckii* subsp. *bulgaricus.*

Phospholipase and chymosin:

Phospholipase A1 (YieldMAX®PL from Chr. Hansen A/S, Denmark; average activity 2600 LEU/ml, dosage 0.021650% for 5% fat)
Bovin chymosin (CHY-MAX®Plus from Chr. Hansen A/S, Denmark; average activity 200 IMCU/ml, dosage 0.00026% for 2% casein) Fat and protein content was determined using MilkoScan analysis.

Table 10 Composition and fermentation details of samples 1-16

| Trial | Homo. (bar) | Starter Culture | Protein | Chymosin (%) | Phospholipase (%) | Ferm. Time | End pH |
|---|---|---|---|---|---|---|---|
| 1 | 150 | 1 | 2.50% | 0 | 0.01265 | 3 h 55 m | 4.47 |
| 2 | 300 | 1 | 2.50% | 0.00026 | 0.01265 | 3 h 55 m | 4.47 |
| 3 | 150 | 2 | 2.50% | 0.00026 | 0.01265 | 4 h 35 m | 4.52 |
| 4 | 300 | 2 | 2.50% | 0 | 0.01265 | 4 h 35 m | 4.54 |
| 5 | 150 | 1 | 4.50% | 0.00046 | 0.01265 | 5 h 30 m | 4.52 |
| 6 | 300 | 1 | 4.50% | 0 | 0.01265 | 5 h 00 m | 4.51 |
| 7 | 150 | 2 | 4.50% | 0 | 0.01265 | 6 h 25 m | 4.56 |
| 8 | 300 | 2 | 4.50% | 0.00046 | 0.01265 | 6 h 25 m | 4.52 |
| 9 | 150 | 1 | 2.50% | 0.00026 | 0 | 4 h 10 m | 4.52 |
| 10 | 300 | 1 | 2.50% | 0 | 0 | 4 h 10 m | 4.52 |
| 11 | 150 | 2 | 2.50% | 0 | 0 | 4 h 20 m | 4.54 |
| 12 | 300 | 2 | 2.50% | 0.00026 | 0 | 4 h 20 m | 4.55 |
| 13 | 150 | 1 | 4.50% | 0 | 0 | 5 h 00 m | 4.54 |
| 14 | 300 | 1 | 4.50% | 0.00046 | 0 | 5 h 00 m | 4.53 |
| 15 | 150 | 2 | 4.50% | 0.00046 | 0 | 6 h 35 m | 4.54 |

(continued)

| Trial | Homo. (bar) | Starter Culture | Protein | Chymosin (%) | Phospholipase (%) | Ferm. Time | End pH |
|---|---|---|---|---|---|---|---|
| 16 | 300 | 2 | 4.50% | 0 | 0 | 6 h 35 m | 4.53 |

Fermentation was carried out at 43°C until a pH of 4.55 ± 0.05 was reached. The fermentation time was recorded. The fermented product was cooled trough a plate heat exchanger post-treated to 23°C - 25°C and smoothed with a back pressure valve at a pressure of 1 bar at the outlet of the cooler.
Samples were stored at cold room temperature (5°C -7°C)
Samples stored at day 7 were tested for viscosity and gel firmness as in Example 1. Results are given below.

Table 11 Rheological assessment of samples at day 7

| | Homo (bar) | Starter Culture | Protein | Chymosin (%) | Phospholipase (%) | Gel Firm. (G*) | Viscosity at 60 1/s | Viscosity at 300 1/s |
|---|---|---|---|---|---|---|---|---|
| 1 | 150 | 1 | 2.50% | 0 | 0.01265 | 129.22 | 425.4 | 97.0 |
| 2 | 300 | 1 | 2.50% | 0.00026 | 0.01265 | 230.46 | 578.0 | 110.9 |
| 3 | 150 | 2 | 2.50% | 0.00026 | 0.01265 | 198.86 | 715.3 | 150.6 |
| 4 | 300 | 2 | 2.50% | 0 | 0.01265 | 156.17 | 633.7 | 168.3 |
| 5 | 150 | 1 | 4.50% | 0.00046 | 0.01265 | 725.84 | 1559.6 | 261.2 |
| 6 | 300 | 1 | 4.50% | 0 | 0.01265 | 531.91 | 1378.3 | 275.3 |
| 7 | 150 | 2 | 4.50% | 0 | 0.01265 | 527.07 | 1745.6 | 375.4 |
| 8 | 300 | 2 | 4.50% | 0.00046 | 0.01265 | 814.83 | 2255.2 | 416.2 |
| 9 | 150 | 1 | 2.50% | 0.00026 | 0 | 173.49 | 441.4 | 88.1 |
| 10 | 300 | 1 | 2.50% | 0 | 0 | 156.15 | 454.4 | 100.1 |
| 11 | 150 | 2 | 2.50% | 0 | 0 | 128.64 | 538.7 | 139.1 |
| 12 | 300 | 2 | 2.50% | 0.00026 | 0 | 218.96 | 726.7 | 154.6 |
| 13 | 150 | 1 | 4.50% | 0 | 0 | 387.86 | 1066.2 | 237.4 |
| 14 | 300 | 1 | 4.50% | 0.00046 | 0 | 779.04 | 1694.5 | 292.9 |
| 15 | 150 | 2 | 4.50% | 0.00046 | 0 | 655.44 | 1816.1 | 354.5 |
| 16 | 300 | 2 | 4.50% | 0 | 0 | 561.71 | 1889.3 | 387.7 |

The rheological responses were analyzed using a stepwise regression analysis with a multilinear model including main effects and first order interactions:

$$\text{Response} = cste + a.F1 + b.F2 + c.F3 + d.F4 + e.F5 + f.F1.F2 + g\ F1.F3...$$

All effects having a risk to mistake above 5% (P-value > 0.05) were eliminated.

Viscosity at 60 1/s

Model Summary:

| S | R-sq | R-sq(adj) | R-sq(pred) |
|---|---|---|---|
| 46.9055 | 99.78% | 99.44% | 98.41% |

The model explains more than 98% of the experimental variation.

Coefficients:

| Term | Coef | SE Coef | T-Value | P-value |
|---|---|---|---|---|
| Constant | 1119.9 | 11.7 | 95.50 | 0.000 |
| Homo. | 81.4 | 11.7 | 6.94 | 0.000 |
| Culture | 170.2 | 11.7 | 14.51 | 0.000 |
| Protein | 555.7 | 11.7 | 47.39 | 0.000 |
| Chymosin | 103.5 | 11.7 | 8.82 | 0.000 |
| Phospholipase | 41.5 | 11.7 | 3.54 | 0.012 |
| Homo.*Protein | 47.4 | 11.7 | 4.04 | 0.007 |
| Homo.*Phospholipase | -31.5 | 11.7 | -2.68 | 0.036 |
| Culture*Protein | 80.8 | 11.7 | 6.89 | 0.000 |
| Protein*Chymosin | 52.3 | 11.7 | 4.46 | 0.004 |

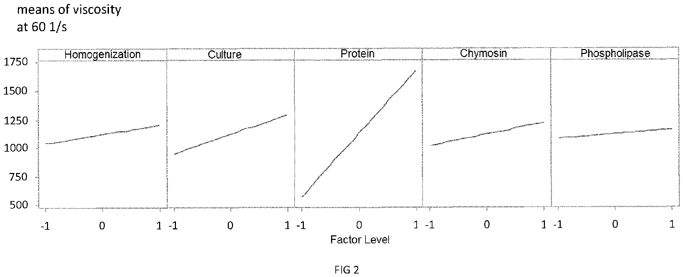

Effects of different factors are plotted in Figure 2.

Viscosity at 300 1/s

Model Summary:

| S | R-sq | R-sq(adj) | R-sq(pred) |
|---|---|---|---|
| 8.81291 | 99.64% | 99.41% | 98.87% |

The model explains more than 98% of the experimental variation.

Coefficients:

| Term | Coef | SE Coef | T-Value | P-value |
|---|---|---|---|---|
| Constant | 225.57 | 2.20 | 102.38 | 0.000 |
| Homo. | 12.68 | 2.20 | 5.75 | 0.000 |
| Culture | 42.71 | 2.20 | 19.39 | 0.000 |
| Protein | 99.49 | 2.20 | 45.16 | 0.000 |
| Phospholipase | 6.28 | 2.20 | 2.85 | 0.019 |
| Homo.*Protein | 5.29 | 2.20 | 2.40 | 0.040 |
| Culture*Protein | 15.67 | 2.20 | 7.11 | 0.000 |

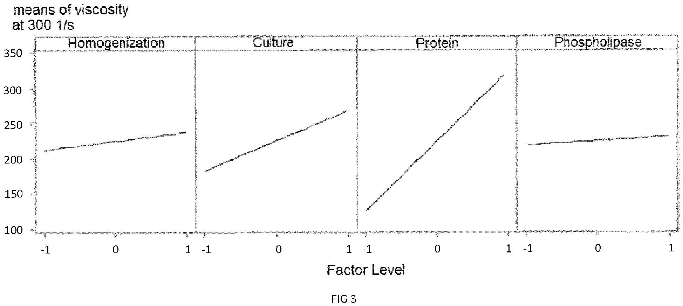

Effects of different factors are plotted in Figure 3.

Gel firmness at day 7 (G*)

Model Summary:

| S | R-sq | R-sq(adj) | R-sq(pred) |
|---|---|---|---|
| 9.66033 | 99.96% | 99.86% | 99.38% |

The model explains more than 99% of the experimental variation.

Coefficients:

| Term | Coef | SE Coef | T-Value | P-value |
|---|---|---|---|---|
| Constant | 398.48 | 2.42 | 165.00 | 0.000 |
| Homo. | 32.68 | 2.42 | 13.53 | 0.000 |
| Culture | 9.23 | 2.42 | 3.82 | 0.019 |
| Protein | 224.48 | 2.42 | 92.95 | 0.000 |
| Chymosin | 76.14 | 2.42 | 31.53 | 0.000 |
| Phospholipase | 15.82 | 2.42 | 6.55 | 0.003 |
| Homo.*Protein | 16.23 | 2.42 | 6.72 | 0.003 |
| Homo.*Phospho. | -13.63 | 2.42 | -5.64 | 0.005 |
| Culture*Protein | 7.57 | 2.42 | 3.13 | 0.035 |
| Culture*Chymosin | -11.82 | 2.42 | -4.90 | 0.008 |
| Protein*Chymosin | 44.69 | 2.42 | 18.50 | 0.000 |
| Protein*Phospho. | 11.13 | 2.42 | 4.61 | 0.010 |

As evidenced from above, impact of phospholipase on the texture measured at both viscosity points was confirmed with high level of significance (p-value = 0.012 and 0.019), showing its positive effect on both mouthfeel and cohesiveness. The effect of phospholipase was higher at a lower homogenization level (significant negative interaction, p-value = 0.036). Maximum viscosity gain is +145 mPas at homogenization level of 150 bars. No interaction was observed with culture; phospholipase appears to work the same regardless of the fermentation kinetic. There was no negative interaction between phospholipase and chymosin, showing that their combined effect is purely additive and maximum gain can reach +457 mPas. This confirms that the addition of chymosin is able to further improve the texture.

Similar strong effects and same conclusions can be drawn for the gel firmness response.

Example 3

Soy yoghurt containing 2.03% fat and 4.05% protein was prepared. Effect of phospholipase on rheological properties at day 7 was evaluated.

Table 12 Composition used for the preparation of soy yoghurt (w/w)

| Ingredient | Specification | Dosage (%) |
|---|---|---|
| Chinese Soy Powder | Redman | 22.50 |
| Sugar | KSL, Refined Sugar | 5.00 |
| Water | City Water, PUB | 72.50 |

Table 13 Samples of Example 3

| Sample | Culture | Dosage | Enzyme | |
|---|---|---|---|---|
| S1 | YF-L DA01 | 200U/MT | - | |
| S2 | YF-L DA01 | 200U/MT | Yieldmax®PL | 0.0046% |

Preparation and Fermentation:

Depending on the setup, phospholipase was added before, at the start or during the fermentation period.

The method used in example 3 was as follows. The composition displayed in table 12 was mixed with a Silverson Mixer. The mixing temperature/hydration time was of 45°C - 50°C /20min. The homogenization was carried out with a GEA Lab Homogenizer with a pre-heat of 65-70°C and the pressure was of 150 bar for the 1st stage and 50 bar for the 2nd stage, amounting to a total of 200 bar. The process was carried out with a Scandinox waterbath, with a pasteurization condition of 85°C / 30 min and a cooling temperature of less than 10°C. The fermentation was carried out in scandinox Waterbath, with a temperature of 43°C and an end pH of 4.55 $\pm$ 0.05.

The post treatment for the soy yoghurt was the following: a pilot treatment unit (PTU) was used with a back pressure of 1.0 bar and a cooling temperature of 23°C -25°C.

The rheology studies of samples obtained from example 3 were carried out as follows. The rheology studies were performed with a Anton Paar Modular Compact Rheometer MCR 302. The measuring temperature was of 13°C and the program used was Stirred oscillation + Up_DownFlow.

Table 14 Fermentation details

| Recipe | S1 | S2 |
|---|---|---|
| End pH | 4.60 | 4.58 |
| Fermentation Time | 9hrs 10mins | 9hrs 10mins |
| pH @ D+7 | 4.52 | 4.53 |

Table 15 Oscillation and hysteresis curve for each sample after a shelf life period of D+7 days

| | | Oscillation | Hysteresis curve | | | |
|---|---|---|---|---|---|---|
| Shelf Life | Sample | G* @ 1.52Hz (pa) | Vis pt 1 | Vis pt 5 | Vis pt 21 | Area |
| D+7 | S1 | 452.43 | 74453 | 1178.8 | 295.65 | 6396.17 |
| | S2 | 537.42 | 86539 | 1342.8 | 317.83 | 7145.82 |

- Vis pt 1: Viscosity (mpa.s) at 0.271 s-1 (initial state, resistance when spoon out the yoghurt)
- Vis pt 5: Viscosity at 60 s-1 (correlate well with texture in mouth)
- Vis pt 21: Viscosity at 300 s-1 (cohesiveness (difficulty to swallow) / mouth thickness)
- Hysteresis Area: correlate to resistance to shear
- Oscillation G* @ 1.52Hz: correlate to Gel Firmness

As evidenced from above for the soy yoghurt, the addition of phospholipase (YieldMAX®PL) showed a positive effect on the texture of the soy yoghurt, with the Vis pt 5 of S2 being 13.9% higher compared to S1.

The sensory evaluation of soy yoghurt showed that S2 was thicker in texture, creamier, and less astringent in taste compared to S1. Thus, the addition of addition of phospholipase (YieldMAX®PL) improves the texture and taste of soy yoghurt.

**Claims**

1. A process for producing a fermented milk product comprising the steps of

   a) adding a starter culture comprising at least one lactic acid bacteria strain to a milk base **characterized by** a fat content of at least 1.5% (w/w),
   b) fermenting the milk base for a period of time until a target pH is reached, wherein the target pH is 5.0 or lower, and
   c) adding at least one phospholipase A to the milk base at the start of the fermentation period.

2. The method of any of the preceding claims, wherein the phospholipase is phospholipase A1 (EC 3.1.1.32).

3. The method of any of the preceding claims, wherein the phospholipase is phospholipase A2 (EC 3.1.1.4).

4. The method of any of the preceding claims, wherein the target pH is 4.6 or lower.

5. The method of any of the preceding claims, wherein the milk base is **characterized by** a fat content of at least 2.0% (w/w).

**6.** The method of any of the preceding claims, wherein the lactic acid bacteria is selected from the group consisting of bacteria from the genera *Lactobacillus, Streptococcus, Lactococcus,* and *Leuconostoc.*

**7.** The method of claim 1, wherein the starter culture contains at least one *Lactobacillus delbrueckii* subsp. *bulgaricus* strain and at least one *Streptococcus thermophilus* strain.

**8.** The method of any one of preceding claims, further comprising adding a chymosin (EC 3.4.23.4) to the milk base before, at the start or during the fermentation period.

**9.** The method of any one of preceding claims, wherein the fermented milk product is selected from the group consisting of quark, cream cheese, fromage frais, sour milk, cultured milk, kefir, lassi, ayran, doogh, yakult, dahi and soy yoghurt.

**10.** The method of any of the preceding claims, wherein the milk base homogenized at 150 bar or less.

**11.** A fermented milk product produced from the method of any of the preceding claims.

**12.** The fermented milk product of claim 11, **characterized in that** the fermented milk product has increased viscosity as compared to the fermented milk product produced without adding phospholipase.

**13.** Use of phospholipase A to increase the viscosity of fermented milk products.

**14.** Use of phospholipase A and chymosin (EC 3.4.23.4) to increase the viscosity of fermented milk products.

**15.** A kit for producing a fermented milk product comprising at least one phospholipase A and a frozen or freeze-dried direct vat set (DVS) starter culture comprising at least one lactic acid bacteria strain.

**Patentansprüche**

**1.** Prozess zum Produzieren eines fermentierten Milchprodukts, umfassend die folgenden Schritte:

a) Hinzugeben einer Starterkultur, die mindestens einen Milchsäurebakterienstamm umfasst, zu einer Milchbasis, die durch einen Fettgehalt von mindestens 1,5 % (Gew.-%) gekennzeichnet ist,
b) Fermentieren der Milchbasis über einen Zeitraum, bis ein Ziel-pH-Wert erreicht ist, wobei der Ziel-pH-Wert 5,0 oder niedriger ist, und
c) Hinzugeben mindestens einer Phospholipase A zu der Milchbasis zu Beginn des Fermentationszeitraums.

**2.** Verfahren nach einem der vorhergehenden Ansprüche, wobei die Phospholipase Phospholipase A1 (EC 3.1.1.32) ist.

**3.** Verfahren nach einem der vorhergehenden Ansprüche, wobei die Phospholipase Phospholipase A2 (EC 3.1.1.4) ist.

**4.** Verfahren nach einem der vorhergehenden Ansprüche, wobei der Ziel-pH-Wert 4,6 oder niedriger ist.

**5.** Verfahren nach einem der vorhergehenden Ansprüche, wobei die Milchbasis durch einen Fettgehalt von mindestens 2,0 % (Gew.-%) gekennzeichnet ist.

**6.** Verfahren nach einem der vorhergehenden Ansprüche, wobei die Milchsäurebakterien aus der Gruppe ausgewählt sind, die aus Bakterien der Gattungen *Lactobacillus, Streptococcus, Lactococcus* und *Leuconostoc* besteht.

**7.** Verfahren nach Anspruch 1, wobei die Starterkultur mindestens einen *Lactobacillus delbrueckii* subsp. *bulgaricus-Stamm* und mindestens einen *Streptococcus thermophilus-Stamm* enthält.

**8.** Verfahren nach einem der vorhergehenden Ansprüche, ferner umfassend Hinzugeben eines Chymosins (EC 3.4.23.4) zu der Milchbasis vor, zu Beginn oder während des Fermentationszeitraums.

**9.** Verfahren nach einem der vorhergehenden Ansprüche, wobei das fermentierte Milchprodukt aus der Gruppe ausgewählt ist, die aus Quark, Rahmkäse, Frischkäse, Dickmilch, Sauermilch, Kefir, Lassi, Ayran, Doogh, Yakult, Dahi und Sojajoghurt besteht.

10. Verfahren nach einem der vorhergehenden Ansprüche, wobei die Milchbasis bei 150 bar oder weniger homogenisiert wird.

11. Fermentiertes Milchprodukt, produziert nach dem Verfahren nach einem der vorhergehenden Ansprüche.

12. Fermentiertes Milchprodukt nach Anspruch 11, **dadurch gekennzeichnet, dass** das fermentierte Milchprodukt verglichen mit dem ohne Hinzufügen von Phospholipase produzierten fermentierten Milchprodukt eine erhöhte Viskosität aufweist.

13. Verwendung von Phospholipase A zum Erhöhen der Viskosität fermentierter Milchprodukte.

14. Verwendung von Phospholipase A und Chymosin (EC 3.4.23.4) zum Erhöhen der Viskosität fermentierter Milchprodukte.

15. Ausrüstung zum Produzieren eines fermentierten Milchprodukts, umfassend mindestens eine Phospholipase A und eine gefrorene oder gefriergetrocknete Direktimpfungs(direct vat set - DVS)-Starterkultur, die mindestens einen Milchsäurebakterienstamm umfasst.

**Revendications**

1. Procédé de production d'un produit de lait fermenté comprenant les étapes

a) d'ajout d'une culture de départ comprenant au moins une souche de bactéries lactiques à une base de lait **caractérisée par** une teneur en matières grasses d'au moins 1,5 % (p/p),
b) de fermentation de la base de lait pendant une période de temps jusqu'à ce qu'un pH cible soit atteint, dans lequel le pH cible est inférieur ou égal à 5,0, et
c) d'ajout d'au moins une phospholipase A à la base de lait au début de la période de fermentation.

2. Procédé selon l'une quelconque des revendications précédentes, dans lequel la phospholipase est la phospholipase A1 (EC 3.1.1.32).

3. Procédé selon l'une quelconque des revendications précédentes, dans lequel la phospholipase est la phospholipase A2 (EC 3.1.1.4).

4. Procédé selon l'une quelconque des revendications précédentes, dans lequel le pH cible est inférieur ou égal à 4, 6.

5. Procédé selon l'une quelconque des revendications précédentes, dans lequel la base de lait est **caractérisée par** une teneur en matières grasses d'au moins 2,0 % (p/p).

6. Procédé selon l'une quelconque des revendications précédentes, dans lequel les bactéries lactiques sont choisies dans le groupe constitué de bactéries des genres *Lactobacillus, Streptococcus, Lactococcus* et *Leuconostoc.*

7. Procédé selon la revendication 1, dans lequel la culture de départ contient au moins une souche de *Lactobacillus delbrueckii* sous-espèce *bulgaricus* et au moins une souche de *Streptococcus thermophilus.*

8. Procédé selon l'une quelconque des revendications précédentes, comprenant en outre l'ajout d'une chymosine (EC 3.4.23.4) à la base de lait avant, au début ou pendant la période de fermentation.

9. Procédé selon l'une quelconque des revendications précédentes, dans lequel le produit de lait fermenté est choisi dans le groupe constitué du fromage blanc, du fromage à la crème, du fromage frais, du lait caillé, du lait de culture, du kéfir, du lassi, de l'ayran, du doogh, du yakult, du dahi et du yaourt au soja.

10. Procédé selon l'une quelconque des revendications précédentes, dans lequel la base de lait est homogénéisée à 150 bars ou moins.

11. Produit de lait fermenté produit à partir du procédé selon l'une quelconque des revendications précédentes.

**12.** Produit de lait fermenté selon la revendication 11, **caractérisé en ce que** le produit de lait fermenté présente une viscosité accrue par rapport au produit de lait fermenté produit sans ajout de phospholipase.

**13.** Utilisation de la phospholipase A pour accroître la viscosité de produits de lait fermentés.

**14.** Utilisation de la phospholipase A et de la chymosine (EC 3.4.23.4) pour accroître la viscosité de produits de lait fermentés.

**15.** Kit destiné à la production d'un produit de lait fermenté comprenant au moins une phospholipase A et une culture de départ congelée ou lyophilisée en cuve directe (DVS) comprenant au moins une souche de bactéries lactiques.

FIG 1

means of viscosity
at 60 1/s

FIG 2

means of viscosity
at 300 1/s

FIG 3

Shear Stress τ [Pa]

FIG 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007095958 A1, Chr. Hansen
- JP S57189638 A
- WO 9826057 A
- WO 0032758 A
- US 4106991 A
- US 4661452 A
- EP 238216 A
- WO 9529999 A
- WO 2002036752 A
- WO 2013174840 A
- WO 2015128417 A

**Non-patent literature cited in the description**

- **CASADO, VICTOR et al.** Phospholipases in food industry: a review. *Lipases and Phospholipases: Methods and Protocols,* 2012, 495-523